(19) Europäisches Patentamt
European Patent Office
Office européen des brevets

(11) **EP 3 501 493 A1**

(12) **EUROPEAN PATENT APPLICATION**
published in accordance with Art. 153(4) EPC

(43) Date of publication:
**26.06.2019 Bulletin 2019/26**

(51) Int Cl.:
*A61K 8/9789* (2017.01)   *A61Q 19/08* (2006.01)

(21) Application number: **17840644.3**

(22) Date of filing: **24.05.2017**

(86) International application number:
**PCT/BR2017/050129**

(87) International publication number:
**WO 2018/032074 (22.02.2018 Gazette 2018/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.08.2016 BR 102016019120**

(71) Applicant: **Natura Cosméticos S.A.**
**05106-000 São Paulo SP (BR)**

(72) Inventors:
• **TADINI D'ANNOLFO, Kassandra**
**05106-000 São Paulo - SP (BR)**

• **CAROLLO MONCAYO, Priscila**
**05106-000 São Paulo - SP (BR)**
• **DE OLIVEIRA REIS, Eduardo Alexandre**
**05106-000 São Paulo - SP (BR)**
• **PAES, Fabiana**
**05106-000 São Paulo - SP (BR)**
• **SANTOS DE OLIVEIRA, Ricardo Augusto**
**05106-000 São Paulo - SP (BR)**
• **ZIMBARDI, Daniela**
**05106-000 São Paulo - SP (BR)**

(74) Representative: **Nicolle, Frederick Joseph**
**Simmons & Simmons LLP**
**CityPoint**
**One Ropemaker Street**
**London EC2Y 9SS (GB)**

(54) **COSMETIC ANTI-BLEMISH COMPOSITION, USE OF THE COMPOSITION, ANTI-BLEMISH TREATMENT METHOD AND APPLICATION DEVICE**

(57)    The present invention relates to super-concentrated cosmetic anti-sign compositions, in the form of an elixir, particularly useful in the treatment of wrinkles, especially in the area under eyes.

FIG. 1

EP 3 501 493 A1

**Description**

**FIELD OF THE INVENTION**

The present invention relates to super-concentrated cosmetic anti-sign compositions, in the form of an elixir, particularly useful in the treatment of wrinkles, especially in the area under eyes.

**BACKGROUND**

Skin aging is the result of several factors. In addition to each individual's genetic predisposition, there are external factors and factors related to life style and quality, where our organism, including the skin, undergoes alterations and slowing down of several physiological processes.

Wrinkles can appear in people of any age and sex and, as ultimate perception, the skin appearance compromised with deep wrinkles end by imparting a fatigue and aging appearance to those people afflicted thereby.

Cosmetic compositions, even if super-concentrated, provably effective in reducing the amount of wrinkles, particularly in the area under the eyes, while improving firmness of the eyelids are still desirable.

**BRIEF DESCRIPTION OF THE FIGURES**

Figure 1 is a graph illustrating average values of Ur/Ue parameters obtained at the beginning of the study, 30 minutes after the first application, and after applying the cosmetic composition according to the present invention at home for 7, 14, 28 and 56 days.

Figure 2 is a graph illustrating average values of Ur/Ue parameters obtained at the beginning of the study, 30 minutes after the first application, and after applying the cosmetic composition according to the present invention at home for 7, 14, 28 and 56 days.

**DESCRIPTION OF THE INVENTION**

The present invention relates to super-concentrated cosmetic anti-sign compositions, in the form of an elixir, which are particularly useful in the treatment of wrinkles, specially in the area under eyes, while improving firmness of the eyelids.

By "elixir", as used herein, it is intended to mean any cosmetic compositions which are super-concentrated in active ingredients targeted to reducing wrinkles and improving firmness of the eyelids. By "super-concentrated" it is intended to mean any cosmetic compositions containing more than about 7% of one or more active compounds, particularly from about 7 to about 15% of one or more active compounds.

The cosmetic anti-sign composition according to the present invention is an elixir comprising:

a) at least one emollient;
b) at least one humectant;
c) at least one active compound;
d) at least one emulsifier;
e) at least one sensorial modifier; and
f) cosmetically acceptable vehicles.

The emollient is selected from the group consisting of capric/caprylic trigliceride, caprylyl methicone, alkyl benzoate having 12 to 15 carbons, dibutyl adipate, dicaprylyl carbonate, isononyl isononanoate, dicapryl ether, dodecane, ethylhexyl palmitate, ethyl macadamiate, isohexadecane, isoamyl cocoate, or mixtures thereof. In a particular embodiment, the emollient is capric/caprylic trigliceride.

The humectant is selected from the group consisting of glycerol, glycols, sorbitol, mannitol, or mixtures thereof. In a particular embodiment, the humectant is glycerol.

The active compound is selected from the group consisting of Hymenaea courbaril extract, Spilanthes Acmella extract, a mixture of silanetriol/hyaluronic acid, a mixture of sodium cocoyl amino acids/sarcosine/potassium aspartate/magnesium aspartate/propylene glycol, acethyl tetrapeptide-2, a mixture of Paeonia albiflora extract / phenoxyethanol / ethylhexylglycerine, extrato de Cichorium intybus, or mixtures thereof. In particularl the active compound is Hymenaea courbaril extract, Spilanthes Acmella extract, a mixture of silanetriol/hyaluronic acid, a mixture of sodium cocoyl amino acids/sarcosine/potassium aspartate/magnesium aspartate/propylene glycol, or mixtures thereof.

The emulsifier is selected from the group consisting of a mixture of cetearyl isononanoate/ ceteareth-20/ cetearyl alcohol / glyceril stearate / glycerine / ceteareth-12 / cethyl palmitate, acrylates, xanthan gum, glyceril citrate, potassium cethylphosphate, PEG-100, cetearyl alcohol, a mixture of glyceril stearate/PEG-100, or mixtures thereof. In particular the emulsifier is a mixture of cetearyl isononanoate/ ceteareth-20/ cetearyl alcohol / glyceril stearate / glycerine / ceteareth-12 / cethyl palmitate, acrylates, xanthan gum, or mixtures thereof. Particularly the emulsifier is cyclopentasiloxane.

The sensorial modifier is selected from the group consisting of cyclopentasiloxane, dimethicone, cyclopentasiloxane/ dimethicone crospolymers/ titanium isopropyl triisostearate, nylon-12, polimethylsilsesquioxane, or mixtures thereof.

The cosmetically acceptable vehicles can be selected from those compounds known in the art.

As examples of vehicles there can be mentioned: solvents, preservatives, perfumes/fragrances, polymer neutralizing agents, chelating agents (such as disodium EDTA), pH adjusting agents, and the like.

The cosmetic compositions according to the present invention act on 11 main mechanisms responsible for wrinkle formation on the area under the eyes and eyelid flaccidity. Those are as follows:

- Hyaluronic acid replacement;
- Skin microtension relaxation;
- Microdamage inhibition;
- Collagen stimulation;
- Elastin stimulation;
- Cell renewal;
- Colllagen protection (anti-glycant action);
- Hyaluronic acid protection;
- Elastin protection;
- Lipid protection; and
- Natural hydration recovery.

This simultaneous action on 11 mechanisms is possible because of the super-concentrate of synergistically and simultaneously acting active ingredients containing:

- Hymenaea courbaril extract: it increases collagen production, while protecting the already existing collagen (anti-glycant action).
- Low molecular weight hyaluronic acid targeted by a technologically treated silanel to improve permeation thereof across the skin. It is the main component responsible for aiding skin filling. Addition thereof to the product causes a filling effect, such that the skin cells are potentiated to produce hyaluronic acid for naturally filling wrinkles, in addition to allow an increase in hydration by impounding water molecules.
- Amino acids (a mixture of sodium cocoyl amino acids/sarcosine/potassium aspartate/magnesium aspartate/propylene glycol, acethyl tetrapeptide-2): those are amino acids which are essential to the skin and enriched by potassium and magnesium having targeting systems containing skin natural constituents. They act on the skin protecting (epidermis) and sustaining (dermis) layer, on the mechanisms of inhibition of the skin sustaining fiber degradation having an anti-elastase and anti-lipoxigenyse action and regulating the damage mechanisms by regulating the IL-6 and IL-8 inflammatory agents.
- Glycerol, specially obtained from palm oil: it is a humectant that binds to water in the environment thereby helping maintaining hydration.

The compositions according to the present invention can be provided in the form of a medicine-dropper to facilitate dropwise application directly onto the skin area to be treated, particularly the area under the eyes.

Therefore, the present invention also contemplates an applicator device which is a medicine-dropper containing the composition of the present invention.

In a particular embodiment, the cosmetic compositions according to the present invention act on the expression signs, in addition to protecting, replacing, and stimulating hyaluronic acid, showing improvement in the eyelid firmness.

Another object of the present invention is the use of the cosmetic compositions in anti-sign treatment, particularly in wrinkle treatment, in the area under the eyes.

Still another object of the present invention is a method of anti-sign treatment, as well as method of wrinkle reduction, particularly in the area under the eyes, consisting of applying a cosmetically effective amount of a composition according to the present invention, on the area under the eyes, at least twice daily. In a particular embodiment, the cosmetically effective amount consists of drops.

The following examples, but without any limitation, illustrate the cosmetic anti-sign compositions according to the present invention, which surprisingly act, simultaneously, on 11 main mechanisms responsible for wrinkle formation in the area under the eyes, and eyelid flaccidity.

**EXAMPLES**

**Example 1. Composition according to the present invention**

The following table illustrates cosmetic compositions according to the present invention.

**Table 1. Cosmetic anti-sign compositions**

| Ingredient | COMPOSITION A | COMPOSITION B |
|---|---|---|
| Alkyl acrylate TR-2 | 0.10 | 0.20 |
| Water | 74.60 | 76.80 |
| Benzyl alcohol / glycerine /chlorphenesin | 1.00 | 1.50 |
| capirc/caprylic triglyceride | 2.50 | 2.00 |
| Cyclopentasiloxane | 4.00 | 3.50 |
| Dissodium EDTA | 0.10 | 0.10 |
| Ethylene/acrylic acid copolymer | 0.50 | 0.70 |
| Eumulgade CM (a mixture of cetearyl isononanoate/ceteareth-20/ cetearyl alcohol /glyceril stearate / glycerine/ ceteareth-12 / cethyl palmitato) | 3.00 | 4.00 |
| Glycerine (glycerol) | 5.00 | 3.00 |
| Hymenaea courbaril | 0.25 | 0.15 |
| Silanetriol / hyaluronic acid | 5.00 | 4.50 |
| a mixture of sodium cocoyl amino acids/sarcosine/potassium aspartate/magnesium aspartate/propylene glycol | 3.00 | 2.50 |
| Spilanthes Acmella | 0.25 | 0.15 |
| Xanthan gum (Keltrol SFT) | 0.70 | 0.90 |
| Total | 100.00 | 100.00 |

**Example 2. Assessment of the increase in firmness and elasticity of the skin using cytometry**

The methodology consisted of assessing the increase in firmness and elasticity of the skin by using cytometry measurements (Cytometer® MPA-580 and Multiprobe Adapter MPA-580, CKeletronics, Germany) taken at the beginning of the study, 30 minutes after the first application of the test product at the laboratory and after applying the test product at home for 7, 14, 28 and 56 days. Cytometry measurements were taken on the forearm area. The increase in firmness of the skin was assessed by means of the Ur/Ue parameter (R5) and the increase in elasticity of the skin was assessed through the Ur/Uf parameter (R7).

Firmness of the skin was assessed through the Ur/Ue parameter. The Ur/Ue parameter is the ratio between instant retraction and instant deformation of the skin and corresponds to the biological elasticity.

An increase in firmness is evidenced by an increase in the Ur/Ue parameter Ur/Ue parameter that reflects an increase in the properties of the elastic fibers and collagen.

Elasticity of the skin was assessed through the Ur/Uf parameter that is the ratio between instant retraction and total deformation of the skin, including the viscous part of the skin deformation, and corresponds to the biological elasticity.

An increase in elasticity of the skin is evidenced by an increase in the Ur/Uf parameters that reflects an increase in the properties of the elastic fibers.

From the average values of the Ur/Ue parameter the percentage increase in firmness of the skin (%AF) is calculated, according to Equation 1.

$$\%AF_{ti} = 100 * (Ur/Ue_{ti} - Ur/Ue_{t0}) / Ur/Ue_{t0} \text{ (Equation 1)}$$

where: %AFti = percentage increase in firmness; Ur/Ueti= average values of the Ur/Ue parameter obtained after day i of the study (i = 30 minutes, 7, 14, 28 and 56 days); Ur/Urt0= average values of the Ur/Ue parameter obtained at the beginning of the study (basal).

From the average values of the Ur/Uf parameter the percentage increase in elasticity of the skin (%AE) is calculated, according to Equation 2.

$$\%AEti = 100 * (Ur/Uf\ ti - Ur/Uf\ t0) / Ur/Uf\ t0 \quad (Equation\ 2)$$

where: %AEti = percentage increase in elasticity; Ur/Ufti= average values of the Ur/Uf parameter obtained after day i of the study (i = 30 minutes, 7, 14, 28 and 56 dias); Ur/Uft0= average values of the Ur/Uf parameter obtained at the beginning of the study (basal).

The significance of the increase in skin firmness is evaluated by applying the Student's bimodal paired t-test, considering a confidence interval of 95%, to the average basal values of the Ur/Ue parameter as compared to the values obtained after a i-day use; i = 30 minutes, 7, 14, 28 and 56 days. A suitable result is reached when Ur/Ueti is significantly greater than Ur/Uet0 (P < 0,05), indicating an increase in firmness of the skin.

The significance of the increase in skin elasticity is evaluated by applying the Student's bimodal paired t-test, considering a confidence interval of 95%, to the average basal values of the Ur/Uf parameter as compared to the values obtained after a i-day use; i = 30 minutes, 7, 14, 28 and 56 dias. A suitable result is reached when Ur/Ufti is significantly greater than Ur/Uf t0 (P < 0,05), indicating an increase in skin elasticity.

25 participants completed the study; average age: 43 $\pm$ 14 years. No adverse reactions were noticed or reported during the study.

The cosmetic composition according to the present invention was applied twice daily, once in the morning and once at night.

1 drop on the forearm area, with movements from inside out until its complete absorption.

According to the results achieved, a significant increase in firmness of the forearm skin could be seen after applying the test product at home for 7, 14, 28 and 56 days.

The percentage values of increase in firmness of the forearm skin were: 3,4% after applying the test product at home for 7 days, 6,4% for 14 days, 9,1% for 28 days and 13,7% for 56, as compared to the initial condition of the skin.

76,0% of the participants showed an increase in firmness of the forearm skin after applying the test product at home for 7 days and 96,0% for 14, 28 and 56 days.

There was a significant increase in elasticity of the forearm skin after applying the test product at home for 7, 14, 28 and 56 days.

The percentage values of increase in elasticity of the forearm skin were: 1,6% after applying the test product at home for 7 days, 3,5% for 14 days, 5,3% for 28 days and 8,8% for 56 days.

76,0% of the participants showed an increase in elasticity of the forearm skin after applying the test product at home for 7 days, 80,0% for 14 days, 92,0% for 28 days and 96,0% for 56 days.

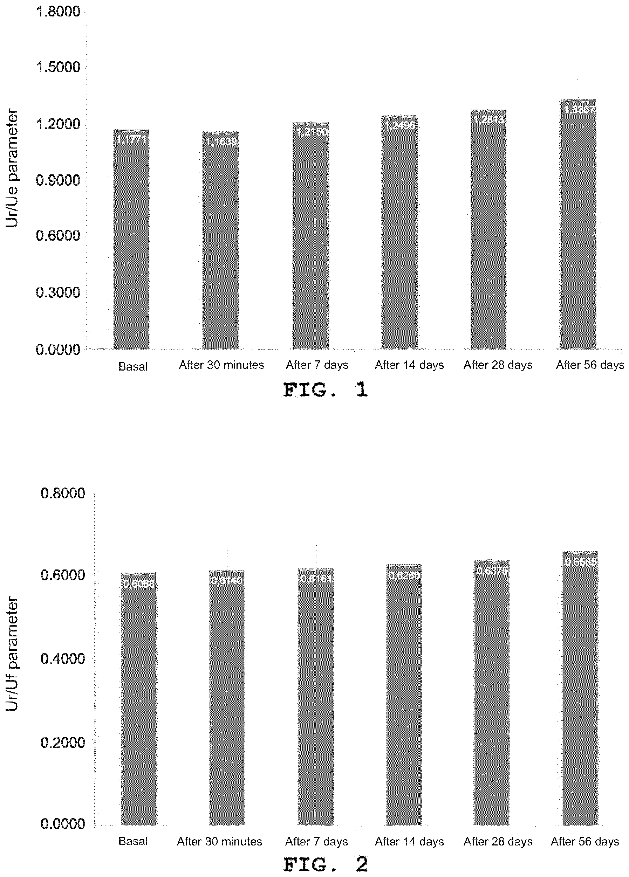

The increase in elasticity of the skin is illustrated on the graph in figure 1, which shows the average values of the Ur/Ue parameter obtained at the beginning of the study, 30 minutes after the first application and after applying the test product at home for 7, 14, 28 and 56 days.

The increase in firmness of the skin is illustrated on the graph in figure 2, which shows the average values of the Ur/Uf parameter obtained at the beginning of the study, 30 minutes after the first application and after applying the test product at home for 7, 14, 28 and 56 days.

**Example 3. Assessment of the efficacy of a cosmetic product through the efficacy perceived by a volunteer in the research, by evaluating the dermatological clinical efficacy, and by using instrumental measurements under normal conditions of use**

The volunteers in the research were evaluated by a dermatologist at the beginning of the study (D0) for the criteria of admission and non-admission and were also evaluated at the end of the study for eventual reactions or discomforts experienced while using the product.

Those volunteers admitted in the research (female individuals, aged 39-70 years - average age 57 years -, having grade II to V wrinkles and I to IV phototype) were clinically evaluated by a dermatologist for the initial condition of the face complexion and subsequently they were advised to fill in a self-evaluation questionnaire also related to the initial condition of the face complexion (D0).

In this step, initial images were also acquired from the periorbital area using the Optical 3D Skin Measuring Device PRIMOS Compact 5.075 equipment. Imaging of the periorbital area was performed at random.

A supervised application of the product was performed on all participants.

Within 30 minutes after the first application of the product, new images were acquired by using the Optical 3D Skin Measuring Device PRIMOS Compact 5.075 equipment. New images were acquired after a 7-, 14-, 28- and 56 day (+/- 2 days)-use of the product.

Further assessments of the perceived and clinical efficacy were performed through questionnaires, 10 minutes after the first application of the products (Dimediato) and after a 7-, 14-, 28- and 56 day (+/- 2 days)-use of the product.

The participants were advised to apply the product at home for 56 days (+/- 2 days), according to the instructions provided.

The participants remained at rest in a room at controlled temperature and relative humidity (20 ° C $\pm$ 2 ° C and 50% $\pm$ 5 RH) for 30 minutes prior to and while the measurements were taken.

- An improvement in firmness of the eyelids 10 minutes after application was seen;
- An improvement in firmness of the eyelids at D14 as compared to D7 was seen;
- An improvement in wrinkles (deep lines), expression lines/signs and firmness of the eyelids at D28 as compared to D7 was seen;
- An improvement in wrinkles (deep lines) and expression lines/signs at D28 as compared to D14 was seen;
- An improvement in wrinkles (deep lines), expression lines/signs, amount of expression lines and firmness of the eyelids at D56 as compared to D7 was seen;
- An improvement in wrinkles (deep lines), expression lines/signs, amount of expression lines and firmness of the eyelids at D56 as compared to D14 was seen;
- An improvement in wrinkles (deep lines) and amount of expression lines at D56 as compared to D28 Dimediato was seen:
- 76% of the volunteers in the research reported an increase in "Firmness of the eyelids";
- 58% of the volunteers in the research reported an improvement in "Wrinkle appearance and Expression lines".

D7:

- 56% of the volunteers in the research reported a reduction in "Wrinkles (deep lines)";
- 55% of the volunteers in the research reported a reduction in "Expression lines/signs";
- 63% of the volunteers in the research reported a reduction in "Amount of expression lines";
- 58% of the volunteers in the research reported an increase in "Firmness of the eyelids".

D14:

- 66% of the volunteers in the research reported a reduction in "Wrinkles (deep lines)";
- 69% of the volunteers in the research reported a reduction in "Expression lines/signs";
- 74% of the volunteers in the research reported a reduction in "Amount of expression lines";
- 69% of the volunteers in the research reported an increase in "Firmness of the eyelids".

D28:

- 77% of the volunteers in the research reported a reduction in "Wrinkles (deep lines)";
- 79% of the volunteers in the research reported a reduction in "Expression lines/signs";
- 71% of the volunteers in the research reported a reduction in "Amount of expression lines";
- 76% of the volunteers in the research reported an increase in "Firmness of the eyelids".

D56:

- 93% of the volunteers in the research reported a reduction in "Wrinkles (deep lines)";
- 92% of the volunteers in the research reported a reduction in "Expression lines/signs";

- 88% of the volunteers in the research reported a reduction in "Amount of expression lines";
- 87% of the volunteers in the research reported an increase in "Firmness of the eyelids".

**Assessment of Clinical Efficacy**

As compared to time point D0:

- An improvement in the feature "Healthy appearance" at time points Dimediate, D7, D14, D28 and D56 as compared to time point D0;
- An improvement in the feature "Firmness of the eyelids" at time points D7, D28 and D56 as compared to time point D0 was seen;
- An improvement in "General appearance" at time points D7, D14, D28 and D56 as compared to time point D0 was seen;
- An improvement in the feature "Expression lines/signs", at time points D28 and D56 as compared to time point D0 was seen;
- An improvement in the features "Extent of wrinkles" and "Amount of lines" at time point D56 as compared to time point D0 was seen.

As compared to time point Dimediato:

- An improvement in the feature "Firmness of the eyelids" at time points D7, D28 and D56 as compared to time point Dimediato was seen;
- An improvement in the features "General appearance" and a "Healthy appearance" at time points D7, D14, D28 and D56 as compared to time point Dimediato was seen;
- An improvement in the feature "Expression lines/signs" at time points D28 and D56 as compared to time point Dimediato was seen;
- An improvement in the features "Extent of wrinkles" and "Amount of lines" at time point D56 as compared to time point Dimediato was seen.

As compared to time point D7:

- A worsening in the feature "Firmness of the eyelids", at time point D14 as compared to time point D7 was seen;
- An improvement in the feature "Firmness of the eyelids", at time point D56 as compared to time point D7 was seen;
- An improvement in the features "General appearance" and "Healthy appearance" at time points D14, D28 and D56 as compared to time point D7;
- An improvement in the feature "Expression lines/signs" at time points D28 and D56 as compared to time point D7 was seen;
- An improvement in the features "Extent of wrinkles" and "Amount of lines" at time point D56 as compared to time point D7.

As compared to time point D14:

- An improvement in the features "Expression lines/signs", "General appearance" and "Healthy appearance" at time points D28 and D56 as compared to time point D14 was seen;
- An improvement in the features "Extent of wrinkles"; "Amount of lines" and "Firmness of the eyelids" at time point D56 as compared to time point D14 was seen.

As compared to time point D28:

- An improvement in the features "Extent of wrinkles", "Expression lines/signs", "Amount of lines", "Firmness of the eyelids", "General appearance" and "Healthy appearance" at time point D56 as compared to time point D28 was seen.

Assessment of cutaneous protuberance - Primos:

- The product promoted a reduction in Volume, Average roughness (Ra), Average deepness (Rz), Maximum roughness (Rm), Waviness (Wt) and Deepness, shortly after application of the product and after a seven-, fourteen-, twenty-eight-, and fifty-six-day use.

The product promoted a reduction in Texture after a seven-, fourteen-, twenty-eight-, and fifty-six-day use.

**Example 4. Assessment of the efficacy of a cosmetic product through the efficacy perceived by a volunteer in the research, by evaluating the dermatological clinical efficacy, and by using instrumental measurements under normal conditions of use**

The volunteers in the research were evaluated by a dermatologist at the beginning of the study (D0) for the criteria of admission and non-admission and were also evaluated at the end of the study for eventual reactions or discomforts experienced while using the product.

Those volunteers admitted in the research (female individuals, aged 39-70 years - average age 57 years -, having grade II to V wrinkles and I to IV phototype) were clinically evaluated by a dermatologist for the initial condition of the face complexion and subsequently they were advised to fill in a self-evaluation questionnaire also related to the initial condition of the face complexion (D0).

In this step, initial images were also acquired from the periorbital area using the Optical 3D Skin Measuring Device PRIMOS Compact 5.075 equipment. Imaging of the periorbital area was performed at random.

A supervised application of the product was performed on all participants.

Within 30 minutes after the first application of the product, new images were acquired by using the Optical 3D Skin Measuring Device PRIMOS Compact 5.075 equipment. New images were acquired after a 7-, 14-, 28- and 56 day (+/- 2 days)-use of the product.

Further assessments of the perceived and clinical efficacy were performed through questionnaires, 10 minutes after the first application of the products (Dimediato) and after a 7-, 14-, 28- and 56 day (+/- 2 days)-use of the product.

The participants were advised to apply the product at home for 56 days (+/- 2 days), according to the instructions provided.

The participants remained at rest in a room at controlled temperature and relative humidity (20 ° C $\pm$ 2 ° C and 50% $\pm$ 5 RH) for 30 minutes prior to and while the measurements were taken.

Assessment of Perceived Efficacy

- An increase in firmness of the eyelids 10 minutes after application was seen;
- An improvement in Firmness of the eyelids at D14 as compared to D7 was seen;
- An improvement in Wrinkles (deep lines), Expression lines/signs and firmness of the eyelids at D28 as compared to D7 was seen;
- An improvement in Wrinkles (deep lines) and Expression lines/signs at D28 as compared to D14 was seen;
- An improvement in Wrinkles (deep lines), Expression lines/signs, amount of expression lines and firmness of the eyelids at D56 as compared to D7 was seen;
- An improvement in Wrinkles (deep lines), Expression lines/signs, amount of expression lines and firmness of the eyelids at D56 as compared to D14 was seen;
- An improvement in Wrinkles (deep lines) and Amount of expression lines at D56 as compared to D28 was seen.

Dimediato:

- 76% of the volunteers in the research reported an increase in "Firmness of the eyelids";
- 58% of the volunteers in the research reported an improvement in "Wrinkle appearance and Expression lines".

D7:

- 56% of the volunteers in the research reported a reduction in "Wrinkles (deep lines)";
- 55% of the volunteers in the research reported a reduction in "Expression lines/signs";
- 63% of the volunteers in the research reported a reduction in "Amount of expression lines";
- 58% of the volunteers in the research reported an increase in "Firmness of the eyelids".

D14:

- 66% of the volunteers in the research reported a reduction in "Wrinkles (deep lines)";
- 69% of the volunteers in the research reported a reduction in Expression lines/signs";
- 74% of the volunteers in the research reported a reduction in "Amount of expression lines";
- 69% of the volunteers in the research reported an increase in "Firmness of the eyelids".

D28:

- 77% of the volunteers in the research reported a reduction in "Wrinkles (deep lines)";
- 79% of the volunteers in the research reported a reduction in "Expression lines/signs";
- 71% of the volunteers in the research reported a reduction in "Amount of expression lines";
- 76% of the volunteers in the research reported an increase in "Firmness of the eyelids".

D56:

- 93% of the volunteers in the research reported a reduction in "Wrinkles (deep lines)";
- 92% of the volunteers in the research reported a reduction in "Expression lines/signs";
- 88% of the volunteers in the research reported a reduction in "Amount of expression lines";
- 87% of the volunteers in the research reported an increase in "Firmness of the eyelids".

**Assessment of Clinical Efficacy**

As compared to time point D0:

- An improvement in the feature "Healthy appearance" at time points Dimediate, D7, D14, D28 and D56 as compared to time point D0 was seen;
- An improvement in the feature "Firmness of the eyelids" at time points D7, D28 and D56 as compared to time point D0 was seen;
- An improvement in "General appearance" at time points D7, D14, D28 and D56 as compared to time point D0 was seen;
- An improvement in the feature "Expression lines/signs", at time points D28 and D56 as compared to time point D0 was seen;
- An improvement in the features "Extent of wrinkles" and "Amount of lines" at time point D56 as compared to time point D0 was seen.

As compared to time point Dimediato:

- An improvement in the feature "Firmness of the eyelids" at time points D7, D28 and D56 as compared to time point Dimediato was seen;
- An improvement in the features "General appearance" and "Healthy appearance" at time points D7, D14, D28 and D56 as compared to time point Dimediato was seen;
- An improvement in the feature "Expression lines/signs" at time points D28 and D56 as compared to time point Dimediato was seen;
- An improvement in the features "Extent of wrinkles" and "Amount of lines" at time point D56 as compared to time point Dimediato was seen.

As compared to time point D7:

- A worsening of the feature "Firmness of the eyelids", at time point D14 as compared to time point D7 was seen;
- An improvement in the feature "Firmness of the eyelids", at time point D56 as compared to time point D7 was seen;
- An improvement in the features "General appearance" and "Healthy appearance" at time points D14, D28 and D56 as compared to time point D7 was seen;
- An improvement in the feature "Expression lines/signs" at time points D28 and D56 as compared to time point D7 was seen;
- An improvement in the features "Extent of wrinkles" and "Amount of lines" at time point D56 as compared to time point D7 was seen.

As compared to time point D14:

- An improvement in the features "Expression lines/signs", "General appearance" and "Healthy appearance" at time points D28 and D56 as compared to time point D14 was seen;
- An improvement in the features "Extent of wrinkles"; "Amount of lines" and "Firmness of the eyelids" at time point D56 as compared to time point D14 was seen.

As compared to time point D28:

- An improvement in the features "Extent of wrinkles", "Expression lines/signs", "Amount of lines", "Firmness of the eyelids", "General appearance" and "Healthy appearance" at time point D56 as compared to time point D28 was seen.

Assessment of cutaneous protuberance - Primos:

- The product promoted a reduction in Volume, Average roughness (Ra), Average deepness (Rz), Maximum roughness (Rm), Waviness (Wt) and Deepness, shortly after application of the product and after a seven-, fourteen-, twenty-eight-, and fifty-six-day use.
- The product promoted a reduction in Texture after a seven-, fourteen-, twenty-eight-, and fifty-six-day use.

Based on the teachings provided in the disclosure of the invention and examples one skilled in the art would be able to appreciate the advantages of the invention and propose variations and alternative equivalent embodiments, without departing from the scope of the invention, as defined in the appended claims.

**Claims**

1.   A COSMETIC ANTI-SIGN COMPOSITION **characterized in that** the composition is an elixir comprising:

   a) at least one emollient;
   b) at least one humectant;
   c) at least one active compound;
   d) at least one emulsifier;
   e) at least one sensorial modifier; and
   f) cosmetically acceptable vehicles.

2.   THE COMPOSITION according to claim 1, **characterized in that** the emollient is selected from the group consisting of capric/caprylic triglyceride, caprylyl methicone, alkyl benzoate having 12 to 15 carbons, dibutyl adipate, dicaprylyl carbonate, isononyl isononanoate, dicapryl ether, dodecane, ethylhexyl palmitate, ethyl macadamiate, isohexadecane, isoamyl cocoate, or mixtures thereof.

3.   THE COMPOSITION according to claim 2, **characterized in that** the emollient is capric/caprylic triglyceride.

4.   THE COMPOSITION according to claim 1, **characterized in that** the humectant is selected from the group consisting of glycerol, glycols, sorbitol, mannitol, or mixtures thereof.

5.   THE COMPOSITION according to claim 4, **characterized in that** the humectant is glycerol.

6.   THE COMPOSITION according to claim 1, **characterized in that** the active compound is selected from the group consisting of Hymenaea courbaril extract, extrato de Spilanthes Acmella, a mixture of silanetriol/hyaluronic acid, a mixture of sodium cocoyl amino acids/sarcosine/potassium aspartate/magnesium aspartate/propylene glycol, acethyl tetrapeptide-2, a mixture of Paeonia albiflora extract/ phenoxyethanol / ethylhexylglycerine, extrato de Cichorium intybus, ou mixtures thereof.

7.   THE COMPOSITION according to claim 6, **characterized in that** the active compound is Hymenaea courbaril extract, Spilanthes Acmella extract, a mixture of silanetriol/hyaluronic acid, a mixture of sodium cocoyl amino acids/sarcosine/potassium aspartate/magnesium aspartate/propylene glycol, ou mixtures thereof.

8.   THE COMPOSITION according to claim 7, **characterized in that** the composition comprises form about 7 to about 15% of one or more active compounds.

9.   THE COMPOSITION according to claim 1, **characterized in that** the emulsifier is selected from the group consisting of a mixture of cetearyl isononanoate/ ceteareth-20/ cetearyl alcohol / glyceril stearate / glycerine / ceteareth-12 / cethyl palmitate, acrylates, xanthan gum, glyceril citrate, potassium cethylphosphate, PEG-100, cetearyl alcohol, a mixture of glyceril stearate/PEG-100, or mixtures thereof.

10.   THE COMPOSITION according to claim 9, **characterized in that** the emulsifier is selected from the group consisting of a mixture of cetearyl isononanoate/ ceteareth-20/ cetearyl alcohol / glyceril stearate / glycerine / ceteareth-12 / cethyl palmitate, acrylates, xanthan gum, or mixtures thereof.

11. THE COMPOSITION according to claim 1, **characterized in that** the sensorial modifier is selected from the group consisting of cyclopentasiloxane, dimethicone, cyclopentasiloxane/ dimethicone crospolymers/ titanium isopropyl triisostearate, nylon-12, polimethylsilsesquioxane, or mixtures thereof.

12. THE COMPOSITION according to claim 11, **characterized in that** the sensorial modifier is cyclopentasiloxane.

13. USE OF THE COMPOSITION, as defined in one of claims 1 to 12, **characterized by** being in the anti-sign treatment.

14. THE USE according to claim 13, **characterized in that** it is for treatment of wrinkles, particularly in the area under the eyes.

15. A METHOD OF ANTI-SIGN TREATMENT **characterized by** consisting of applying a cosmetically effective amount of the composition as defined in one of claims 1 to 12, in the area under the eyes, at least twice daily.

16. THE METHOD according to claim 15, **characterized in that** it is used in the reduction in wrinkles in the area under the eyes.

17. AN APPLICATOR DEVICE, **characterized in that** the device is a medicine-dropper comprising the composition as defined in one of claims 1 to 12.

FIG. 1

FIG. 2

## INTERNATIONAL SEARCH REPORT

| International application No. |
| --- |
| PCT/BR2017/050129 |

**A. CLASSIFICATION OF SUBJECT MATTER**

**A61K 8/9789 (2017.01), A61Q 19/08 (2006.01)**

According to International Patent Classification (IPC) or to both national classification and IPC

**B. FIELDS SEARCHED**

Minimum documentation searched (classification system followed by classification symbols)

**A61K 8/9789 , A61Q 19/08**

Documentation searched other than minimum documentation to the extent that such documents are included in the fields searched

**INPI-BR Banco de Patentes (SINPI)**

Electronic data base consulted during the international search (name of data base and, where practicable, search terms used)

**Google Patent, Espacenet, EPODOC**

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2015031971 A2 (NATURA COSMÁTICOS S A [BR])<br>12 March 2015 (2015-03-12)<br>The whole document | 1<br>2 to 17 |
| Y | EP 0326491 A1 ( BONNE CLAUDE [FR])<br>02 August 1989 (1989-08-02)<br>The whole document | 1 to 17 |
| Y | JP 2001322940 A (KAO CORP)<br>20 November 2001 (2001-11-20)<br>The whole document | 1 to 17 |
| Y | JP 2003055190 A (KOEI PERFUMERY)<br>26 February 2003 (2003-02-26)<br>The whole document | 1 to 17 |

☐ Further documents are listed in the continuation of Box C.    ☒ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **10/08/2017** | **17/08/2017** |

| Name and mailing address of the ISA/ **BR** | Authorized officer |
| --- | --- |
| INSTITUTO NACIONAL DA PROPRIEDADE INDUSTRIAL<br>Rua Sao Bento nº 1, 17º andar<br>cep: 20090-010, Centro - Rio de Janeiro/RJ | **Rodrigo Barbosa Ferraro** |
| Facsimile No.    +55 21 3037-3663 | Telephone No.    +55 21 3037-3984/3742 |

Form PCT/ISA/210 (second sheet) (January 2015)

INTERNATIONAL SEARCH REPORT
Information on patent family members

International application No.

PCT/BR2017/050129

| | | | |
|---|---|---|---|
| WO 2015031971 A2 | 2015-03-12 | WO 2015031971 A8 | 2015-07-09 |
| | | AR 097599 A1 | 2016-03-23 |
| | | AU 2014317742 A1 | 2016-04-21 |
| | | EP 3038715 A2 | 2016-07-06 |
| | | FR 3010313 A1 | 2015-03-13 |
| | | MX 2016003091 A | 2016-08-19 |
| | | US 2016220476 A1 | 2016-08-04 |
| EP 0326491 A1 | 1989-08-02 | EP 0326491 B1 | 1993-11-03 |
| | | AR 240015 A1 | 1990-01-31 |
| | | AT 96655 T | 1993-11-15 |
| | | AU 2936089 A | 1989-08-25 |
| | | AU 611762 B2 | 1991-06-20 |
| | | BR 8905293 A | 1990-08-14 |
| | | CA 1331446 C | 1994-08-16 |
| | | DE 68910435 D1 | 1993-12-09 |
| | | DK 466789 A | 1989-09-21 |
| | | EP 0356472 A1 | 1990-03-07 |
| | | ES 2045479 T3 | 1994-01-16 |
| | | FI 894476 A | 1989-09-21 |
| | | FI 91934 B | 1994-05-31 |
| | | FR 2626469 A1 | 1989-08-04 |
| | | IE 890280 L | 1989-07-29 |
| | | IE 62973 B1 | 1995-03-08 |
| | | JP H02500442 A | 1990-02-15 |
| | | JP H054364 B2 | 1993-01-19 |
| | | KR 920003574 B1 | 1992-05-04 |
| | | MX 172298 B | 1993-12-13 |
| | | NO 893815 A | 1989-09-26 |
| | | NO 174915 B | 1994-04-25 |
| | | NZ 227760 A | 1991-08-27 |
| | | PT 89547 A | 1989-10-04 |
| | | US 4992264 A | 1991-02-12 |
| | | WO 8906954 A1 | 1989-08-10 |
| | | ZA 8900667 B | 1989-09-27 |
| JP 2001322940 A | 2001-11-20 | None | |
| JP 2003055190 A | 2003-02-26 | None | |

Form PCT/ISA/210 (patent family annex) (January 2015)

14